# NOUVEAU FASCICULE DE BREVET EUROPEEN

(11) **EP 0 192 986 B2**
(45) Date de publication et mention de la décision concernant l'opposition: **09.12.1998**
(45) Mention de la délivrance du brevet: 16.08.1989
(21) Numéro de dépôt: 86101202.9
(22) Date de dépôt: 30.01.1986
(51) Int. Cl.: C12N 1/20, A23C 9/123

(54) **Culture de bifidobactéries résistantes à l'acide**
Säurebeständige Bifidobakterienkultur
Acid-resistant bifidus bacteria culture

(30) Priorité: 28.02.1985 CH 916/85
(43) Date de publication de la demande: 03.09.1986
(73) Titulaire: SOCIETE DES PRODUITS NESTLE S.A., 1800 Vevey (CH)
(72) Inventeur: Sozzi, Tomaso, CH-1010 Lausanne (CH)

(56) Documents cités:
- EP-A- 0 112 020
- FR-A- 2 336 886
- NL-A- 7 614 632
- US-A- 4 091 117
- FOOD SCIENCE & TECHNOLOGY ABSTRACTS, no. 81 03 P0528 (81016236), 1981; J. ROSSI: "Low temperature survival of some lactic acid bacteria in lactic beverages", & MICROBIOL. SYMPOSIUM, Instituto di Microbiol. Lattiero Caesaria Dell'Univ. di Perugia, IT, 195-205, 1979
- FOOD SCIENCE & TECHNOLOGY ABSTRACTS, no. 83 01 P0103 (83003984), 1983; Z. ZBIKOWSKI: "Study of use of Bifidobacterium bifidum and Lactobacillus acidophilus in yoghurt production", & ZESZYTY NAUKOWE AKADEMII ROLNICZO TECHNICZNEJ W. OLSZTYNIE, TECHNOLOGIA ZYWNOSCI, 16, 3-73, 1981
- FOOD SCIENCE & TECHNOLOGY ABSTRACTS, no. 84 02 P0291 (84010616), 1982; S. SHIMANURA: "Milk products containing viable Bifidobacteria", & JAPANESE JOURNAL OF DAIRY AND FOOD SCIENCE, 31(6), A245-A253, 1982
- FICHIER JAPIO, 83 224685, 1983; & JP - A - 58 224 685 (MORINAGA NIYUUGIYOU K.K.) 27-12-1983
- FICHIER JAPIO, 82 099190, 1982; & JP - A - 57 099 190 (MIDORI JUJI K.K.) 19-06-1982
- The American Type Culture Collection, Catalogue of Strains I, 13th Edition, 1978, p. 46

## Description

La présente invention a pour objet un procédé de préparation d'une culture de bifidobactéries résistantes à l'acide, une utilisation de cette culture pour la préparation de produits alimentaires contenant des bifidobactéries résistantes à l'acide et les cultures nouvelles bifidobacterium bifidum CNCM I-373 et bifidobacterium breve CNCM I-374.

Les bifidobactéries sont connues comme témoins, voire comme garants d'une flore intestinale saine chez le nourrisson. Leur usage sous forme viable dans un but diététique, notamment dans des produits du type laits acidifiés, p.ex. des yogourts, se heurte cependant à des difficultés connues telles que leur sensibilité à l'oxygène et à l'acide. Quelques souches ont déjà été proposées qui présentent une bonne résistance à l'oxygène, voire même une résistance à l'acide non négligeable. Cependant, les bifidobactéries les plus résistantes connues ne survivent guère plus de quelques jours, voire une semaine aux pH présentés par des laits acidifiés tels que p.ex. des yogourts au cours de leur conservation sous réfrigération.

Le but de la présente invention est de fournir des cultures de bifidobactéries résistantes à l'acide qui survivent longtemps dans des produits alimentaires tels que des laits acidifiés, notamment des yogourts.

A cet effet, le procédé selon la présente invention est caractérisé par le fait que l'on cultive en milieu lactique, semi-synthétique ou synthétique une souche de bifidobactéries présentant un taux de survie d'au moins 1:10 après 30 j à pH 4,0 à 5°C dans le groupe formé par Bifidobacterium infantis CNCM I-372, Bifidobacterium bifidum CNCM I-373, Bifidobacterium breve CNCM 1-374.

De même, l'utilisation de la culture obtenue par le procédé selon la présente invention est caractérisée par le fait que l'on ajoute ladite culture à un produit alimentaire au cours de sa préparation.

On a constaté en effet qu'il est possible de trouver des souches de bifidobactéries dont la résistance à l'acide est tout à fait surprenante en comparaison de ce qui était connu ou admis jusqu'ici et que l'on peut en préparer des cultures utilisables dans la préparation de produits alimentaires présentant un pH acide tels que des laits acidifiés p.ex. dans lesquels elles présentent un taux de survie remarquable.

Dans le présent exposé, l'expression le «taux de survie» est utilisé pour désigner le nombre de germes survivants dans une culture ou un produit alimentaire après une période de conservation ou après un traitement particulier, par rapport au nombre initial de germes vivants dans la culture ou le produit. Le taux de survie est exprimé par une proportion, p.ex. 1:10.

De même, l'expression «milieu lactique» est utilisée pour désigner un milieu à base essentielle de lait animal ou végétal, entier ou écrémé. L'expression «milieu semi-synthétique» désigne un milieu à base de lait animal ou végétal que l'on a supplémenté par une quantité substantielle d'éléments nutritifs ou de facteurs de croissance pour les bifidobactéries. L'expression «milieu synthétique» désigne un milieu constitué pour l'essentiel par la réunion d'éléments nutritifs et de facteurs de croissance de natures diverses, tel que p.ex. le milieu MRS bien connu de l'homme du métier.

Enfin, le dénombrement des germes de bifidobactéries présentant des difficultés pratiques lorsqu'ils sont mélangés à des germes de streptocoques, ce qui est notamment le cas lorsque les bifidobactéries sont utilisées dans la préparation de produits alimentaires du type laits acidifiés, une méthode particulière de dénombrement des germes de bifidobactéries a été développée et utilisée dans le cadre de la présente invention. Elle est présentée ci-aprés, juste avant les exemples.

Pour mettre en oeuvre le procédé selon la présente invention, on utilise donc un milieu lactique, semi-synthétique ou synthétique. On peut utiliser avantageusement un lait de vache entier ou écrémé auquel on peut ajouter environ 0,1-2% en poids d'extrait de levure et 0,1-2% en poids de glucose, un lait de soja entier ou dégraissé, à savoir un extrait aqueux de fèves entières ou de flocons dégraissés, ou un milieu synthétique tel que le milieu MRS p.ex. Après avoir pasteurisé ou stérilisé le milieu, on peut l'inoculer avec environ 3-10% en volume d'un inoculum contenant par cm³ environ 10⁸-10¹⁰ germes d'une souche de bifidobactéries présentant donc un taux de survie d'au moins 1:10 après 30 j à pH 4,0 à 5°C.

On choisit ladite souche dans le groupe formé par Bifidobacterium, infantis CNCM I-372, Bifidobacterium bifidum CNCM I-373 et Bifidobacterium breve CNCM I-374. Ces 3 souches ont été déposées le 21 novembre 1984 en vertu du Traité de Budapest à la Collection Nationale de Cultures de Microorganismes (CNCM) de l'Institut Pasteur, 25 rue du Docteur Roux, 75724-Paris Cedex 15, France où elles ont donc reçu les Nos 1-372, 1-373 et 1-374. Elles se distinguent non seulement par leur remarquable résistance à l'acide, mais également par une résistance à l'oxygène suffisante pour que le présent procédé puisse être mis en oeuvre et la présente utilisation réalisée sans anaérobiose stricte. Elles présentent enfin ces qualités de résistance à l'oxygène et à l'acide à des degrés semblables. De même, leurs caractéristiques générales, telles que notamment leurs vitesses de croissance sont semblables. Ces similarités font que des cultures de ces 3 souches sont interchangeables dans le cadre de la présente utilisation. Elles peuvent donc être utilisées selon un système de rotation traditionnel destiné à limiter les risques de destruction des bifidobactéries par des phages associés.

On cultive de préférence ladite souche à une température favorable au développement des bifidobactéries, à savoir une température comprise entre environ 35 et 42 °C, durant le temps nécessaire pour que le nombre de germes dans la culture croisse jusqu'à environ 10⁸-10¹⁰/cm³, à savoir durant environ 8-16 h.

Pour réaliser l'utilisation de la culture obtenue selon la présente invention, on peut ajouter ladite culture à un produit alimentaire au cours de sa préparation, lors d'une étape de fermentation ou lors d'une étape de supplémentation par exemple, selon que l'on désire acidifier ledit produit alimentaire avec l'aide de ladite culture elle-même ou que l'on préfère acidifier le produit alimentaire avant d'y ajouter la culture, p.ex.

C'est ainsi que dans une forme d'utilisation préférée, on ajoute ladite culture à un lait pasteurisé ou stérilisé, animal ou végétal, et que l'on acidifie le lait jusqu'à un pH de 4,0-4,6 par fermentation avec ladite culture. On peut ajouter la culture au lait à raison de environ 3-10% en volume et laisser la fermentation se dérouler durant environ 8-16 h à 35-42°C. On obtient ainsi des laits acidifiés riches en bifidobactéries résistantes à l'acide dont la viscosité peut être ajustée en variant la teneur en matière sèche du lait de départ.

Dans une autre forme d'exécution préférée, on ajoute ladite culture à un lait acidifié présentant un pH de 4,0-4,6. Dans cette forme d'exécution, le lait, animal ou végétal, peut avoir été acidifié après pasteurisation ou stérilisation par addition d'acide lactique p.ex. ou par fermentation à l'aide de ferments lactiques tels que des cultures de yogourt p.ex. De cette manière on obtient des laits acidifiés riches en bifidobactéries résistantes à l'acide dont la viscosité peut varier entre celle d'une boisson aqueuse et celle d'un yogourt brassé, selon la teneur en matière sèche du lait de départ.

Les exemples ci-après sont présentés à titre d'illustration de la présente invention. Ils sont précédés d'une description de la méthode particulière de dénombrement des germes de bifidobactéries développée et utilisée dans le cadre de la présente invention.

### Méthode de dénombrement

Une méthode traditionnelle de dénombrement des germes consiste à prélever un échantillon de culture ou de produit à examiner, diluer l'échantillon en milieu aqueux, cultiver une fraction de l'échantillon dilué sur un milieu agar MRS en conditions anaérobies, dénombrer les colonies issues des germes sur l'agar et calculer le nombre original en fonction du facteur de dilution.

Cependant, les bifidobactéries sont le plus souvent mêlées à un grand nombre, voire un surnombre de streptocoques lorsqu'elles sont utilisées dans la préparation de produits alimentaires du type laits acidifiés. Or, les germes de bifidobactéries donnent naissance sur l'agar MRS à des colonies gris-blanches très difficiles à distinguer des colonies issues des germes de streptocoques qui sont de même taille mais blanches. Si l'on applique la méthode traditionnelle ci-dessus au dénombrement des germes de bifidobactéries mêlés à des germes de streptocoques, il est nécessaire de recourir au microscope et il n'est de toute manière pas possible de dénombrer les bifidobactéries dès que leur nombre est inférieur de 2 à 3 puissances de dix au nombre des streptocoques.

C'est ainsi que l'on a développé une méthode particulière qui permette de dénombrer de manière facile et sûre les germes de bifidobactéries, quel que soit le nombre de germes de streptocoques auxquels elles sont mêlées dans l'échantillon à examiner.

La présente méthode consiste à prélever un échantillon de culture ou de produit à examiner, diluer l'échantillon en milieu aqueux, cultiver en conditions anaérobies une fraction de l'échantillon dilué sur un milieu agar MRS modifié en y ajoutant de la nafcilline, à raison de 0,1 µg de nafcilline par cm³ de milieu, dénombrer les colonies issues des germes sur l'agar et calculer leur nombre original en fonction du facteur de dilution.

On a constaté en effet que, à une concentration de environ 0,02-0,1 µg par cm³ de milieu, la nafcilline inhibe complètement la croissance des germes de streptocoques ainsi que de Lactobacillus bulgaricus mais qu'elle n'a aucune influence mesurable sur la croissance des germes de bifidobactéries.

### Exemple 1

A 3 litres de lait écrémé de vache on ajoute 1% en poids d'extrait de levure et 1% en poids de glucose. On pasteurise le lait à 90°C durant 30 min et on le refroidit à 37°C. On l'inocule avec 5% en volume d'un inoculum contenant par cm³ environ 10⁹ germes vivants de Bifidobacterium infantis CNCM I-372 et l'on incube durant 10 h à 37°C On obtient une culture contenant 5×10⁹ germes de B. infantis vivants par cm³.

On ajoute 0,8% en volume de la culture ainsi obtenue à un lait de vache écrémé, pasteurisé et acidifié à pH 4,0 avec de l'acide lactique. On conserve ce lait au réfrigérateur à 5°C et l'on dénombre les germes de B. infantis vivants qu'il contient après 0 j, 7 j et 40 j. On obtient les nombres respectifs de 3,80×10⁷, 3,67×10⁷ et 2,59×10⁷ par cm³ de lait acidifié. En d'autres termes, le taux de survie de la souche B. infantis CNCM 1-372 est pratiquement de 10:10 après 7 j et d'environ 7:10 après 40 j à pH 4,0 à 5 °C.

### Exemple 2

On procède de la manière décrite à l'exemple 1, à l'exception du fait que l'on cultive Bifidobacterium bifidum CNCM I-373 au lieu de B. infantis I-372.

On obtient une culture contenant 1,0×10⁹ germes de B. bifidum vivants par cm³.

Les essais de conservation en lait acidifié à pH 4,0 à 5°C permettent de dénombrer 0,76×10⁷, 0,58×10⁷ et 0,46×10⁷ germes de B. bifidum vivants par cm³ de lait acidifié après respectivement 0 j, 7 j et 40 j. En d'autres termes, le taux de survie de la souche B. bifidum CNCM I-373 est donc d'environ 3:4 après 7 j et 6:10 après 40 j à pH 4,0 à 5°C.

### Exemple 3

On procède de la manière décrite à l'exemple 1 à l'exception du fait que l'on cultive Bifidobacterium breve CNCM I-374 au lieu de B. infantis I-372.

On obtient une culture contenant 1,2×10⁹ germes de B. breve vivants par cm³.

Les essais de conservation en lait acidifié à pH 4,0 à 5°C permettent de dénombrer 0,87×10⁷, 0,62×10⁷ et 0,12×10⁷ germes de B. breve vivants par cm³ de lait acidifié après respectivement 0 j, 7 j et 40 j. En d'autres termes, le taux de survie de la souche B. breve CNCM I-374 est de environ 7:10 après 7 j et environ 14:100 après 40 j à pH 4,0 à 5°C.

### Exemple comparatif

On prépare une culture contenant 4×10⁹ germes de Bifidobacterium infantis CNCM I-372 de la manière décrite à l'exemple 1. De manière semblable on prépare des cultures de B. longum (souche BI 1) et B. breve (souche Bbr 2) normales contenant respectivement 2×10⁹ et 1×10⁹ germes vivants par cm³.

On ajoute 0,8% en volume de ces diverses cultures à des lots distincts de yogourts que l'on conserve ensuite au réfrigérateur à 5°C. On dénombre les germes de bifidobactéries contenues dans ces yogourts après 0 j, 7 j et 21 j. On détermine également le pH de ces yogourts. On obtient les résultats regroupés dans le tableau ci-après qui illustre la mauvaise résistance à l'acide de cultures de bifidobactéries normales comparée à la résistance à l'acide de cultures selon la présente invention.

| Conservation à 5°C j | B. longum (normal, BI 1) | | B. breve (normal, Bbr 2) | | B. infantis (CNCM I-372) | |
|---|---|---|---|---|---|---|
| | pH | germes vivants/cm³ | pH | germes vivants/cm³ | pH | germes vivants/cm³ |
| 0 | 4,28 | 1,6×10⁷ | 4,09 | 7,5×10⁶ | 4,12 | 2,9×10⁷ |
| 7 | 4,22 | 2,8×10⁶ | 4,10 | 2,0×10³ | 4,07 | 2,5×10⁷ |
| 21 | 4,20 | < 10³ | 4,06 | < 10³ | 4,05 | 3,6×10⁷ |

### Exemple 4

On pasteurise à 90°C durant 30 min 100 litres de lait de vache présentant une teneur en matière grasse de 3,5% en poids. On les refroidit à 45°C. On les inocule avec 2% en volume d'une culture de Lactobacillus bulgaricus et 2% en volume d'une culture de Streptococcus thermophilus et on les incube durant 3 h à 45°C. On obtient ainsi environ 100 litres de yogourt présentant un pH de 4,5. On les refroidit à 20°C et on leur ajoute en brassant doucement 3 litres d'une culture de bifidobactéries résistantes à l'acide préparée de la manière décrite à l'exemple 1.

On obtient ainsi un yogourt présentant la viscosité d'un yogourt brassé et contenant environ 10⁸ germes de B. infantis vivants par cm³. On conditionne ce yogourt en pots de 0,15 litres et on le conserve au réfrigérateur à 5°C pendant cinq semaines. Après cette période de conservation, on dénombre plus de 10⁷ germes de B. infantis vivants par cm³ de yogourt.

### Exemple 5

On pasteurise 100 litres de lait écrémé de vache pendant 30 min et on les refroidit à 32°C. On les inocule avec 1% en volume d'un mélange de ferments lactiques mésophiles (S. cremoris, S. lactis, S. lactis déacétylactis, Leuconostoc) et l'on incube 16 h à 30°C.

On obtient ainsi environ 100 litres d'un lait acidifié à pH 4,4 présentant la viscosité d'une boisson épaisse du genre babeurre. On leur ajoute 5 litres d'une culture de bifidobactéries préparées de la manière décrite à l'exemple 2.

On obtient ainsi une boisson lactique acide épaisse contenant plus de 10⁷ germes de B. bifidum vivants par cm³. Après conditionnement en bouteilles et après une période de conservation de cinq semaines au réfrigérateur à 8°C, on dénombre également plus de 10⁷ germes de B. bifidum vivants par cm³ de boisson.

### Exemple 6

On homogénéise 100 litres de lait entier de vache. On les pasteurise à 155°C durant 2 s et on les refroidit à 40°C. On les acidifie jusqu'à pH 4,2 par addition d'acide lactique.

On obtient ainsi 100 litres de lait caillé. On leur ajoute en brassant doucement 5 litres d'une culture de bifidobactéries préparée de la manière décrite à l'exemple 3.

On obtient ainsi un lait caillé présentant la viscosité d'un yogourt brassé et contenant 8×10⁷ germes de B. breve vivants par cm³. Après conditionnement en pots et après une période de conservation de cinq semaines au réfrigérateur à 5°C on dénombre encore 2×10⁷ germes de B. breve vivants par cm³ de lait caillé.

### Exemple 7

On stérilise à 150°C durant 5 s 100 litres de lait de soja contenant 8% en poids de matière sèche de fève de soja entière. On les refroidit à 37°C et on leur ajoute en brassant bien 5 litres d'une culture de bifidobactéries préparée de la manière décrite à l'exemple 2. On incube durant 10 h à 37°C et l'on refroidit à 10°C.

On obtient ainsi un «yogourt» de soja présentant un pH de 4,5 et contenant 6×10⁹ germes de B. bifidum vivants par cm³. Après une période de conservation de cinq semaines à 5°C, on dénombre encore 2×10⁹ germes de B. bifidum vivants par cm³ de ce «yogourt» végétal.

### Exemple 8

On pasteurise un lait entier de vache à 90°C pendant 30 min. On le refroidit à 37°C et on lui ajoute 10% en volume d'une culture de bifidobactéries obtenue de la manière décrite à l'exemple 3. On incube durant 12 h à 37°C et l'on refroidit à 10°C.

On obtient ainsi un «yogourt» présentant un pH de 4,5 et contenant 5×10⁹ germes de B. breve vivants par cm³. Après une période de conservation de cinq semaines à 5°C, on dénombre encore 2×10⁹ germes de B. breve vivants par cm³ de ce «yogourt».

NB: Les souches utilisées pour comparaison à l'exemple comparatif ci-dessus sont dites normales parce qu'elles présentent des résistances à l'acide semblables à celles présentées pratiquement par le 99% des souches de bifidobactéries. BI 1 et Bbr 2 sont les références d'identification de ces souches dans la propre collection du déposant des souches CNCM I-372-374.

## Revendications

1. Procédé de préparation d'une culture de bifidobactéries résistantes à l'acide, caractérisé par le fait que l'on cultive en milieu lactique, semi-synthétique ou synthétique une souche de bifidobactéries présentant un taux de survie d'au moins 1:10 après 30 j à pH 4,0 à 5°C dans le groupe formé par Bifidobacterium infantis CNCM I-372, Bifidobacterium bifidum CNCM I-373 et Bifidobacterium breve CNCM I-374.

2. Culture de bifidobactéries résistantes à l'acide choisie dans le groupe formé par Bifidobacterium bifidum CNCM I-373 et Bifidobacterium breve CNCM I-374, obtenue par le procédé selon la revendication 1.

3. Utilisation de la culture obtenue par le procédé selon la revendication 1 pour la préparation de produits alimentaires contenant des bifidobactéries résistantes à l'acide, caractérisée par le fait que l'on ajoute ladite culture à un produit alimentaire au cours de sa préparation.

4. Utilisation selon la revendication 3 caractérisée par le fait que l'on ajoute ladite culture lors d'une étape de fermentation.

5. Utilisation selon la revendication 4, caractérisée par le fait que l'on ajoute ladite culture à un lait pasteurisé ou stérilisé, animal ou végétal, et que l'on acidifie le lait jusqu'à un pH de 4,0-4,6 par fermentation avec ladite culture.

6. Utilisation selon la revendication 3, caractérisée par le fait que l'on ajoute ladite culture lors d'une étape de supplémentation.

7. Utilisation selon la revendication 6, caractérisée par le fait que l'on ajoute ladite culture à un lait acidifié présentant un pH de 4,0-4,6.

## Claims

1. Process for preparing a culture of acid-resistant bifidobacteria, characterized in that a strain of bifidobacteria having a survival rate of at least 1:10 after 30 days at pH 4.0 and 5°C, in the group consisting of Bifidobacterium infantis CNCM I-372, Bifidobacterium bifidum CNCM I-373 and Bifidobacterium breve CNCM I-374, is cultivated in a semi-synthetic or synthetic lactic medium.

2. Culture of acid-resistant bifidobacteria selected from the group consisting of Bifidobacterium bifidum CNCM I-373 and Bifidobacterium breve CNCM I-374, obtained by the process according to claim 1.

3. Use of the culture obtained by the process according to claim 1 for preparing food products containing acid-resistant bifidobacteria, characterized in that the said culture is added to a food product during its preparation.

4. Use according to claim 3, characterized in that the said culture is added during a fermentation step.

5. Use according to claim 4, characterized in that the said culture is added to a pasteurized or sterilized animal or vegetable milk, and in that the milk is acidified to a pH of 4.0 to 4.6 by fermentation with the said culture.

6. Use according to claim 3, characterized in that the said culture is added during a supplementation step.

7. Use according to claim 6, characterized in that the said culture is added to an acidified milk having a pH of 4.0 to 4.6.

## Patentansprüche

1. Verfahren zur Herstellung einer säurebeständigen Bifidobakterienkultur, dadurch gekennzeichnet, daß man in einem halbsynthetischen oder synthetischen Milchmilieu einen Bifidobakterienstamm züchtet, der nach 30 Tagen bei einem pH-Wert von 4,0 bei 5°C einen Überlebensgrad von wenigstens 1:10 aufweist, ausgewählt aus der aus Bifidobacterium infantis CNCM I-372, Bifidobacterium bifidum CNCM I-373 und Bifidobacterium breve CNCM I-374 gebildeten Gruppe.

2. Säurebeständige Bifidobakterienkultur, ausgewählt aus der aus Bifidobacterium bifidum CNCM I-373 und Bifidobacterium breve CNCM I-374 gebildeten Gruppe, erhalten nach dem Verfahren gemäß Anspruch 1.

3. Verwendung der nach dem Verfahren gemäß Anspruch 1 erhaltenen Kultur zur Herstellung von Nahrungsmittelprodukten, die säurebeständige Bifidobakterien enthalten, dadurch gekennzeichnet, daß man die genannte Kultur zu einem Nahrungsmittelprodukt im Zuge seiner Herstellung zusetzt.

4. Verwendung nach Anspruch 3, dadurch gekennzeichnet, daß man die genannte Kultur im Laufe einer Fermentationsstufe zusetzt.

5. Verwendung nach Anspruch 4, dadurch gekennzeichnet, daß man die genannte Kultur zu einer tierischen oder pflanzlichen, pasteurisierten oder sterilisierten Milch zusetzt und daß man die Milch durch Fermentation mit der genannten Kultur bis auf einen pH-Wert von 4,0 bis 4,6 ansäuert.

6. Verwendung nach Anspruch 3, dadurch gekennzeichnet, daß man die genannte Kultur im Laufe einer Supplementierungsstufe zusetzt.

7. Verwendung nach Anspruch 6, dadurch gekennzeichnet, daß man die genannte Kultur zu einer angesäuerten Milch zusetzt, die einen pH-Wert von 4,0 bis 4,6 aufweist.
